# EUROPEAN PATENT SPECIFICATION

(11) **EP 4 265 210 B1**
(45) Date of publication and mention of the grant of the patent: **09.12.2026**
(21) Application number: 23168941.5
(22) Date of filing: 20.04.2023
(51) Int. Cl.: A61B 18/14

(54) **COLLAPSIBLE ELECTRODE APPARATUS FOR DIAGNOSIS OF ARRHYTHMIAS**
ZUSAMMENKLAPPBARE ELEKTRODENVORRICHTUNG ZUR DIAGNOSE VON ARRHYTHMIEN
APPAREIL A ELECTRODES REPLIABLES POUR LE DIAGNOSTIC D'ARYTHMIES

(30) Priority: 21.04.2022 US 202263333263 P; 16.03.2023 US 202318185265
(43) Date of publication of application: 25.10.2023
(73) Proprietor: BIOSENSE WEBSTER (ISRAEL) LTD., 2066717 Yokneam (IL)
(72) Inventor: RODRIGUEZ SOTO, Juan, 92618, Irvine (US)
(74) Representative: Carpmaels & Ransford LLP

(56) References cited:
- EP-A1- 3 915 477
- US-A1- 2016 143 588
- US-A1- 2018 042 667
- US-A1- 2018 116 539
- US-A1- 2020 345 262
- US-A1- 2021 369 339
- US-A1- 2022 054 198

## Description

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to provide high-density signal maps through the use of several electrodes sensing electrical activity of tissue in an area on the order of a square centimeter. For mapping within an atria or a ventricle (for example, an apex of a ventricle), it is desirable for a catheter to collect larger amounts of data signals within shorter time spans. It is also desirable for such a catheter to be adaptable to different tissue surfaces, for example, flat, curved, irregular or nonplanar surface tissue and be collapsible for atraumatic advancement and withdrawal through a patient's vasculature. The disclosure of US2021/369339A1 provides an apparatus that includes an end effector having loop members with electrodes thereon and is usable with catheter-based systems to measure or provide electrical signals.
The disclosure of EP3915477A1 provides an apparatus that includes an end effector having loop members with electrodes thereon and is usable with catheter-based systems to measure or provide electrical signals.
The disclosure of US2020/345262A1 provides a catheter with an end effector having densely arrayed electrodes on three loops which are arrayed in various planar configurations when the end effector is unconstrained.
The disclosure of US2016/143588A1 provides a catheter adapted or high density mapping and/or ablation of tissue surface that has a distal electrode matrix having a plurality of spines arranged in parallel configuration on which a multitude of electrodes are carried in a grid formation

### SUMMARY OF THE INVENTION

The invention provides an apparatus according to claim 1. Further embodiments are defined in the dependent claims. Disclosed surgical methods are not encompassed by the wording of the claims but are considered as useful for understanding the invention.

### SUMMARY OF THE DISCLOSURE

Example apparatuses disclosed herein are generally usable with catheter-based systems to measure or provide electrical signals within the heart and surrounding vasculature. Example apparatuses generally include an end effector having loop members with electrodes thereon. The loop members are shaped to be delivered through, deployed from, and retracted into a catheter such that the loop members can be collapsed down to fit within the catheter and resiliently spread to form a paddle shape with deployed. Some of the loop members can include features approximate a distal end of the respective loop member to facilitate collapse from the paddle shape when the end effector is retracted into the catheter.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of a catheter having an end effector at a distal portion of the catheter and a proximal handle at a proximal portion of the catheter according to aspects of the present invention.
FIG. 2 is an illustration of a first example support frame assembly of the end effector in an unconstrained configuration according to aspects of the present invention.
FIG. 3 is an illustration of a second example support frame assembly of the end effector in an unconstrained configuration according to aspects of the present invention.
FIG. 4 is an illustration of a third example support frame assembly of the end effector in an unconstrained configuration according to aspects of the present invention.
FIG. 5 is an illustration of a fourth example support frame assembly of the end effector in an unconstrained configuration according to aspects of the present invention.
FIG. 6 is an illustration of a fifth example support frame assembly of the end effector in an unconstrained configuration according to aspects of the present invention.
FIGs. 7A and 7B are illustrations of a side and perspective view of a sixth example end effector configuration according to aspects of the present invention.
FIG. 8 is an illustration of a seventh example support frame assembly of the end effector in an unconstrained configuration according to aspects of the present invention.
FIG. 9 is an illustration of an eighth example support frame assembly of the end effector in an unconstrained configuration according to aspects of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention, which is defined by the claims. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

As used herein, the terms "patient," "host," "user," and "subject" refer to any human or animal subject and are not intended to limit the systems or methods to human use, although use of the subject invention in a human patient represents a preferred embodiment.

The term "proximal" indicates a location closer to the operator whereas "distal" indicates a location further away to the operator or physician.

When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention, which is defined by the claims.

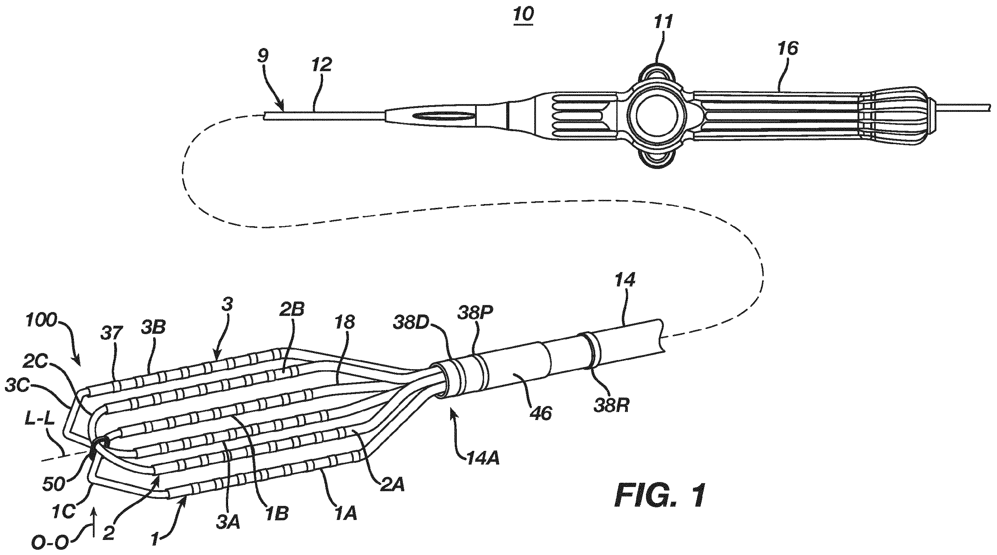

FIG. 1 is an illustration of a catheter apparatus 10 having an end effector 100 at a distal portion of the catheter 10, a proximal handle 16 at a proximal portion of the catheter 10, and an elongated shaft 9 extending between the handle 16 and end effector 100. The shaft 9 is preferably a tubular member. The apparatus 10 can have several design variations, including novel aspects illustrated herein, and other compatible features as understood by a person skilled in the pertinent art. The apparatus 10 is presented for illustration purposes only and is not intended to be limiting.

The elongated shaft 9 has a proximal portion 12 in the shape of an elongated catheter body, an intermediate deflection section 14, and distal portion 14A. The deflection control handle 16 is attached to the proximal end of the proximal portion 12 of the catheter body. The distal portion 14A of the shaft is coupled to the end effector 100 via a connector tubing 46. The elongated shaft 9 forms a tubular catheter body sized and otherwise configured to traverse vasculature. The end effector 100 has a plurality of loop members 1, 2, 3 that overlap at a common distal vertex 50 and can be joined at the distal vertex with a mechanical linkage.

When the device is unconstrained and aligned, the proximal portion 12, intermediate section 14, distal portion 14A, and end effector 100 are generally aligned along a longitudinal axis L-L. The intermediate section 14 can be configured to bend to deflect the distal portion 14A and end effector 100 from the longitudinal axis L-L.

The end effector 100 can be collapsed (compressed toward the longitudinal axis L-L) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA). The sheath is preferably 10 French.

The end effector 100 has first, second and third loop members 1, 2, and 3. Each loop member 1, 2, 3 has two spines 1A, 1B, 2A, 2B, 3A, 3B and a connector 1C, 2C, 3C that connects the two spines of the respective loop member 1, 2, 3. Spines 1A, 1B of a first loop member 1 are connected by a first connector 1C; spines 2A, 2B of a second loop member 2 are connected by a second connector 2C; and spines 3A, 3B of a third loop member 3 are connected by a third connector 3C.

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in FIG. 1. Preferably, all spines in the end effector are parallel to each other along the majority of their respective lengths when the end effector 100 is in the unconstrained configuration. The spines 1A, 1B, 2A, 2B, 3A, 3B can be at least approximately coplanar in the unconstrained configuration. When the end effector 100 is pressed to a planar surface, a majority of electrodes 37 on each spine 1A, 1B, 2A, 2B, 3A, 3B can make contact with the planar surface.

Each spine 1A, 1B, 2A, 2B, 3A or 3B can have a length ranging between about 5 and 50 mm, preferably about 10 and 35 mm, and more preferably about 28 mm. The parallel portions of each spine 1A, 1B, 2A, 2B, 3A, 3B can be spaced apart from each other by a distance ranging between about 1mm and 20 mm, preferably about 2 and 10 mm, and more preferably about 4 mm. Each spine 1A, 1B, 2A, 2B, 3A, 3B can carry at least four electrodes per spine member. As a non-limiting example, each spine can carry between four electrodes per spine member and 10 electrodes per spine member. For example, as illustrated in FIG. 1, each spine 1A, 1B, 2A, 2B, 3A, 3B can carry about eight electrodes per spine member. The end effector can include at least two spines. As non-limiting examples, the end effector can include between two spines and twelve spines. For example, as illustrated in FIG. 1, the end effector can include six spines. With eight electrodes on six spines, for example, the end effector 100 can include forty-eight electrodes.

Each spine 1A, 1B, 2A, 2B, 3A, 3B can include a tubular housing having support frames extending therethrough. Electrodes 37 can be disposed over the tubular housings. Each spine 1A, 1B, 2A, 2B, 3A, 3B can include electrical conductors each electrically connected to a respective electrode 37. The electrical conductors can extend through at least a portion of the respective tubular housing. Some or all of the tubular housings can include a respective irrigation lumen therethrough and irrigation ports.

The catheter 10 can include at least one pull wire extending through the elongated shaft 9 and attached approximate a distal portion 14A of the elongated shaft 9 so that when the pull wire is retracted toward the proximal portion relative to the elongated shaft 9, the distal portion 14A and the end effector 100 are bent at an angle with respect to the longitudinal axis L-L.

A distal electrode 38D and a proximal electrode 38P are positioned near the distal portion 14A of the shaft 9. The electrodes 38D and 38P can be configured to cooperate (e.g. by masking of a portion of one electrode and masking a different portion on the other electrode) to define a referential electrode (an electrode that is not in contact with tissues). One or more impedance sensing electrodes 38R can be configured to allow for location sensing via impedance location sensing technique, as described in US Patent Nos. 5,944,022 and 5,983,126 attached in the Appendix of priority provisional patent application US 63/333,263.

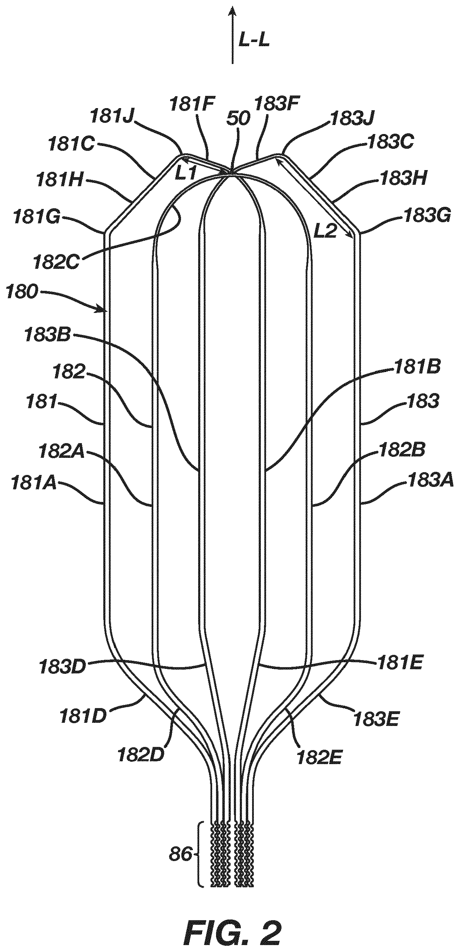

FIG. 2 is an illustration of a first example support frame assembly180 of the end effector 100 in an unconstrained configuration. When the end effector 100 is assembled, the loop members 1, 2, 3 each includes a respective support frame 181, 182, 183. The support frame assembly 180 extends into the connector tubing 46 to mechanically affix the loop members 1, 2, 3 to the shaft 9. The support frames 181, 182, 183 provide structural integrity for the loop members 1, 2, 3. The support frames 181, 182, 183 can include plastic or metal cut-off sheets, plastic or metal round wire, plastic or metal square wire, or other suitable biocompatible material. In the preferred embodiments, the support frames are made from shape memory material such as, for example, nitinol. The support frames 181, 182, 183 overlap at a common distal vertex 50. In the assembled end effector 100, the support frames 181, 182, 183 can be assembled by virtue of a mechanical linkage affixed to an outer housing of the loop members 1, 2, 3 or a direct linkage between the support frames 181, 182, 183.

When the end effector is unconstrained, each of the respective support frames 181, 182, 183 defines a respective looped path of its respective loop member 1, 2, 3. Each support frame 181, 182, 183 includes respective parallel segments 181A, 182A, 183A, 181B, 182B, 183B that extend through corresponding spines 1A, 2A, 3A, 1B, 2B, 3B of the end effector 100. Each support frame 181, 182, 183 includes respective proximal segments 181D, 182D, 183D, 181E, 182E, 183E that extend through corresponding proximal segments 1D, 2D, 3D, 1E, 2E, 3E of respective loop members 1, 2, 3. The proximal segments 181D, 182D, 183D, 181E, 182E, 183E extend into the connector tubing 46 to join the end effector 100 to the shaft 9. Each support frame 181, 182, 183 includes a respective connecting member 181C, 182C, 183C that extends between the respective pair of spines, parallel segments 181A, 182A, 183A, 181B, 182B, 183B and through the respective connecting segment 1C, 2C, 3C of the respective loop member 1, 2, 3.

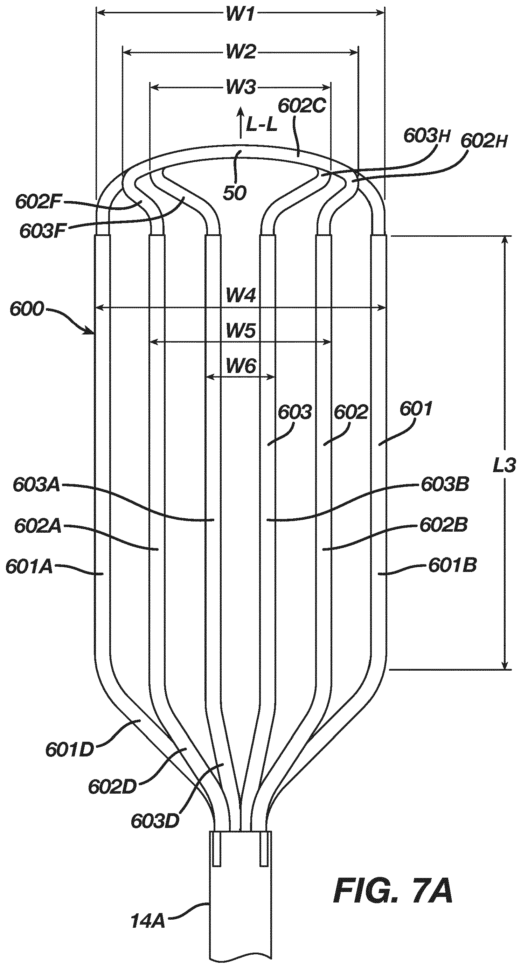

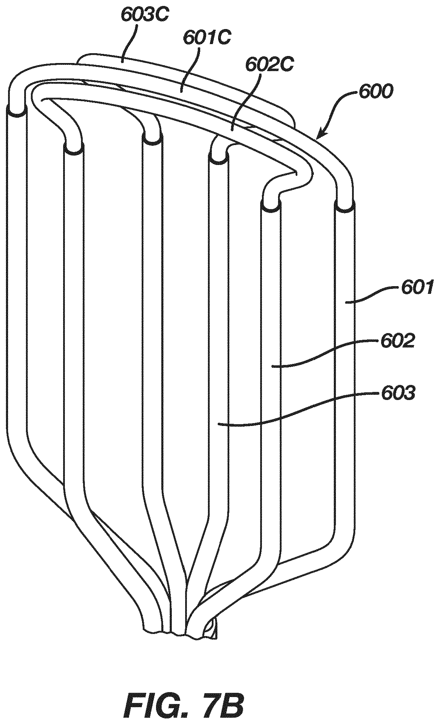

The foregoing description of the support frame assembly 180 also generally describes other example support frame assemblies 280, 380, 480, 580, 780, 880 illustrated in FIGs. 2-6, 8, and 9 as well as a support frame assembly (not illustrated) of the end effector configuration 600 illustrated in FIGs. 7A and 7B. Each example support frame assembly disclosed herein includes connecting segments shaped to facilitate collapse of the end effector 100 into a sheath (preferably a 10 French sheath).

The example support frame assembly 180 illustrated in FIG. 2 shows each of the three connecting members 181C, 182C, 183C joining distal ends of two respective parallel segments 181A, 182A, 183A, 181B, 182B, 183B of the six spines such that a single spine of the six spines is positioned between each of the two respective spines. A first connecting member 181C joins to distal ends of a first spine 181A and a second spine 181B such that the second spine 181B is between two spines 183B, 182B. A second connecting member 182C joins to distal ends of a first parallel segment 182A and a second parallel segment 182B such that the first parallel segment 182A is between two parallel segments 181A, 183B and the second parallel segment 182B is between two parallel segments 181B, 183A. A third connecting member 183C joins distal ends of a first parallel segment 183A and a second parallel segment 183B such that the second parallel segment 183B is between two parallel segments 182A, 181B.

The three connecting members 181C, 182C, 183C include a central connecting member 182C that is approximately symmetric to the longitudinal axis L-L and two outer connecting members 181C, 183C asymmetric to the longitudinal axis L-L. A central support frame 182 includes the central connecting member 182C. Two outer support frames 181, 183 include the two outer connecting members 181C, 183C. A central support member 182 includes the central connecting member 182C.

The central connecting member 182C has an arcuate shape.

Each of the two outer connecting members 181C, 183C include a respective pair of substantially straight segments 181F, 181H, 183F, 183H connected by a respective bend 181J, 183J. The bends 181J, 183J are configured to flex as the end effector is moved between the collapsed, constrained configuration and the unconstrained configuration such that the bends 181J, 183J are more acute in the constrained configuration compared to the unconstrained configuration.

Each of the respective pair of substantially straight segments 181F, 181H, 183F, 183H of the outer connecting members 181C, 183C respectively include a first straight segment 181F, 183F and a second straight segment 181H, 183H. The first straight segment 181F, 183F has a first length L1 measured from the distal vertex 50 to the respective bend 181J, 183J, and the second straight segment 181H, 183H has a second length L2 measured from the bend 181J, 183J to the distal end of the respective parallel segment 181A, 183A. The first length L1 is less than the second length L2.

The outer support frame 181, 183 each further include a respective bend 181G, 183G at the distal end of the respective parallel segment 181A, 183A joined to the second straight segment 181H, 183H of the respective outer connecting member 181C, 183C. These bends 181G, 183G are more obtuse in the constrained configuration compared to the unconstrained configuration.

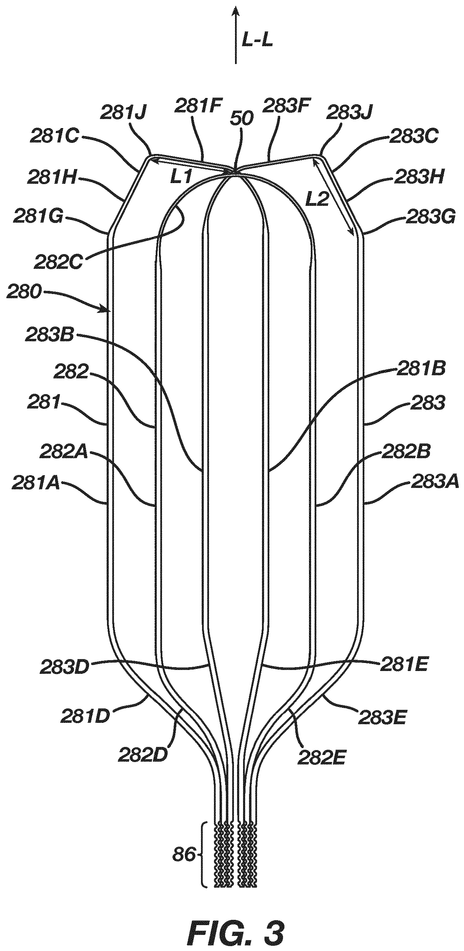

FIG. 3 is an illustration of a second example support frame assembly 280 of the end effector 100 in an unconstrained configuration. The second example support frame assembly 280 includes two outer support frames 281, 283 and a central support frame 282. Each of the support frames 281, 282, 283 includes respective parallel segments 281A, 282A, 283A, 281B, 282B, 283B and respective proximal segments 281D, 282D, 283D, 281E, 282E, 283E configured identically to corresponding features of the first example support frame assembly 180 illustrated in FIG. 2.

Each support frame 281, 282, 283 of the second example support frame assembly 280 includes respective connecting members 281C, 282C, 283C that extend between the respective pair of spines 281A, 282A, 283A, 281B, 282B, 283B. Similar to the first example support frame assembly 180 illustrated in FIG. 2, each of the two outer connecting members 281C, 283C include a respective pair of substantially straight segments 281F, 281H, 283F, 283H connected by a respective bend 281J, 283J that is configured to flex as the end effector is moved between the collapsed, constrained configuration and the unconstrained configuration such that the bends 281J, 283J are more acute in the constrained configuration compared to the unconstrained configuration. However, unlike the first example support frame assembly 180 illustrated in FIG. 2, the length L1 of the first substantially straight segment 282F is approximately equal to the length L2 of the second substantially straight segment for the second example support frame assembly 280 illustrated in FIG. 3.

The second example support frame assembly 280 also includes bends 281G, 283G each between a respective outer spine 281A, 283A and a respective second substantially straight segment 281H, 283H of the respective outer connecting member 281C, 283C configured similarly to corresponding bends 181G, 183G of the first example support frame assembly 180 illustrated in FIG. 2.

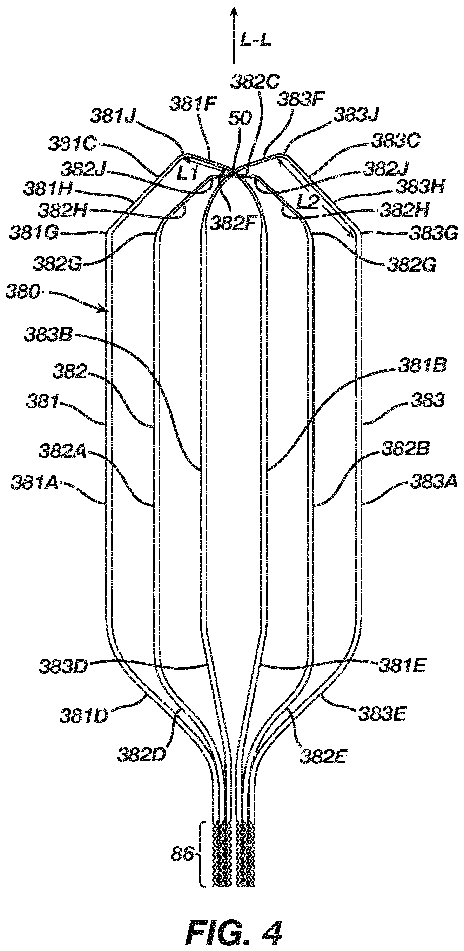

FIG. 4 is an illustration of a third example support frame assembly 380 of the end effector 100 in an unconstrained configuration. The third example support frame assembly 380 includes two outer support frames 381, 383 and a central support frame 382. Each of the support frames 381, 382, 383 includes respective parallel segments 381A, 382A, 383A, 381B, 382B, 383B and respective proximal segments 381D, 382D, 383D, 381E, 382E, 383E configured identically to corresponding features of the first example support frame assembly 180 illustrated in FIG. 2.

Each support frame 381, 382, 383 of the third example support frame assembly 380 includes respective connecting members 381C, 382C, 383C that extend between the respective pair of spines 381A, 382A, 383A, 381B, 382B, 383B. Similar to the first example support frame assembly 180 illustrated in FIG. 2, each of the two outer connecting members 381C, 383C include a respective pair of substantially straight segments 381F, 381H, 383F, 383H connected by a respective bend 381J, 383J that is configured to flex as the end effector is moved between the collapsed, constrained configuration and the unconstrained configuration such that the bends 381J, 383J are more acute in the constrained configuration compared to the unconstrained configuration. Similar to the first example support frame assembly 180 illustrated in FIG. 2, the length L1 of the first substantially straight segment 382F is less than the length L2 of the second substantially straight segment for the third example support frame assembly 380 illustrated in FIG. 4.

The third example support frame assembly 380 also includes bends 381G, 383G each between a respective outer spine 381A, 383A and a respective second substantially straight segment 381H, 383H of the respective outer connecting member 381C, 383C configured similarly to corresponding bends 181G, 183G of the first example support frame assembly 180 illustrated in FIG. 2.

The central connecting member 382C includes two substantially straight angled segments 382H that are angled with respect to the longitudinal axis L-L in the unconstrained configuration and an orthogonal segment 382F that is orthogonal to the longitudinal axis L-L. The central connecting member 382C includes two bends 382J each between the orthogonal segment 382F and a respective angled segment 382H. The bends 382J are configured to become less obtuse as the end effector 100 moves from the unconstrained configuration to the constrained configuration. The central support frame 382 further includes bends 382G respectively positioned at distal ends of the spines 382A, 382B that become more obtuse as the end effector 100 moves from the unconstrained configuration to the constrained configuration.

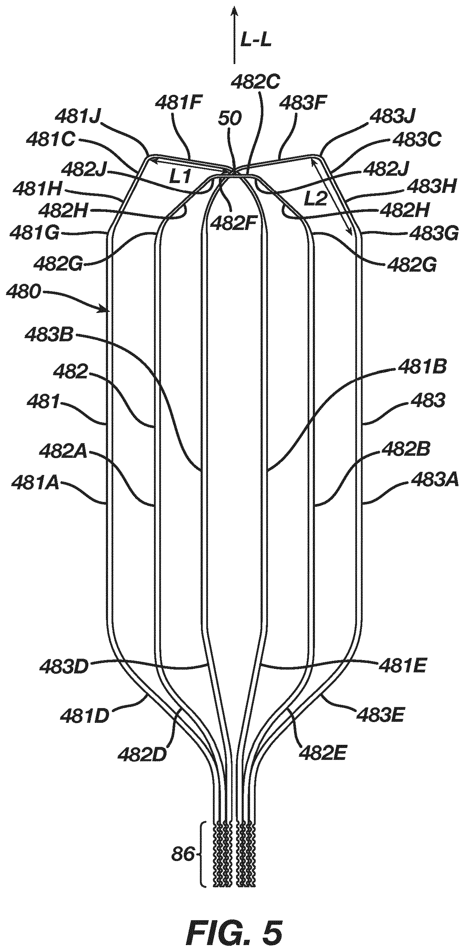

FIG. 5 is an illustration of a fourth example support frame assembly 480 of the end effector 100 in an unconstrained configuration. Each of the support frames 481, 482, 483 includes respective parallel segments 481A, 482A, 483A, 481B, 482B, 483B and respective proximal segments 481D, 482D, 483D, 481E, 482E, 483E configured similarly to corresponding features of the first, second, and third example support frame assemblies 180, 280, 380 illustrated in FIGs. 2 through 4.

The fourth example support frame assembly 480 includes two outer support frames 481, 483 configured similarly to the outer support frames 281, 283 of the second support frame assembly 280 illustrated in FIG. 3. The outer support frames 481, 483 include connecting members 481C, 483C each having two substantially straight segments 481F, 481H, 483F, 483H connected by a respective bend 481J, 483J. The outer support frames 481, 483 further include bends 481G, 483G between the outer spines 481A, 483A and outer connecting members 481C, 483C.

The fourth example support frame assembly 480 includes a central support frame 482 configured similarly to the central support frame 382 of the third example support frame assembly 380 illustrated in FIG. 4. The central support frame 482 includes a central connecting member 482C having two angled segments 482H and an orthogonal segment 482F with bends 482J between the angled segments 482H and the orthogonal segment 482F. The central support frame 482 further includes bends 482G between the angled segments 482H and spines 482A, 482B of the central support frame 482.

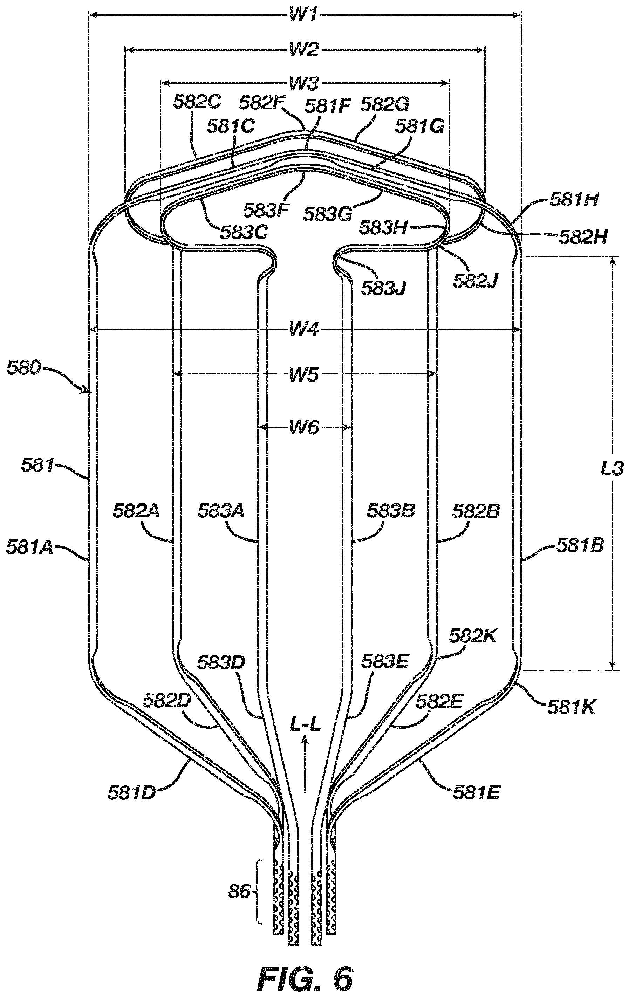

FIG. 6 is an illustration of a fifth example support frame assembly 580 of the end effector 100 in an unconstrained configuration. When the end effector 100 is assembled using the fifth example support frame assembly, the loop members 1, 2, 3 each includes a respective support frame 581, 582, 583. The support frame assembly 580 extends into the connector tubing 46 to mechanically affix the loop members 1, 2, 3 to the shaft 9. The support frames 581, 582, 583 provide structural integrity for the loop members 1, 2, 3. The support frames 581, 582, 583 can include plastic or metal cut-off sheets, plastic or metal round wire, plastic or metal square wire, or other suitable biocompatible material. In the preferred embodiments, the support frames are made from shape memory material such as, for example, nitinol. The support frames 581, 582, 583 overlap at a common distal vertex 50. In the assembled end effector 100, the support frames 581, 582, 583 can be assembled by virtue of a mechanical linkage affixed to an outer housing of the loop members 1, 2, 3 or a direct linkage between the support frames 581, 582, 583.

Note that, when the end effector 100 is assembled using the fifth example support frame assembly, the loop members 1, 2, 3 are each symmetric about the longitudinal axis such that the end effector 100 includes an outer loop member, an inner loop member, and a central loop member supported a first support frame 581, a second support frame 581, and a third support frame 583 respectively.

The outer loop member includes a first pair of proximal segments supported by corresponding proximal segments 581D, 581E of the first support frame 581 and extending distally from the distal portion of the elongated shaft 14 and away from the longitudinal axis L-L, a first pair of spines supported by corresponding parallel segments 581A, 581B of the first support frame 581 and extending distally from the first pair of proximal segments and parallel to the longitudinal axis L-L, and a first connecting member supported by a corresponding connecting member 581C of the first support frame 581 and joining distal ends of the first pair of spines and extending across the longitudinal axis L-L.

The inner loop member includes a second pair of proximal segments supported by corresponding proximal segments 582D, 582E of the second support frame 582 and extending distally from the distal portion of the elongated shaft 14 and away from the longitudinal axis L-L, a second pair of spines supported by corresponding parallel segments 582A, 582B of the second support frame 582 and extending distally from the second pair of proximal segments and parallel to the longitudinal axis L-L, and a second connecting member supported by a corresponding connecting member 582C of the second support frame 582 and joining distal ends of the second pair of spines and extending across the longitudinal axis L-L. The second pair of spines of the inner loop member are positioned between the first pair of spines of the outer loop member.

The central loop member includes a third pair of proximal segments supported by corresponding proximal segments 583D, 583E of the third support frame 583 and extending distally from the distal portion of the elongated shaft 14 and away from the longitudinal axis L-L, a third pair of spines supported by corresponding parallel segments 583A, 583B of the third support frame 583 and extending distally from the third pair of proximal segments and parallel to the longitudinal axis L-L, and a third connecting member supported by a corresponding connecting member 583C of the third support frame 583 and joining distal ends of the third pair of spines and extending across the longitudinal axis L-L. The third pair of spines of the central loop member are positioned between the second pair of spines of the inner loop member.

The connecting members 581C, 582C, 583C are shaped to allow the end effector to collapse to a low profile. The first connecting member 581C includes a pair of substantially straight segments 581G that join at a distal bend 581F. The first support frame 581 includes a pair of bends 581H between the parallel segments 581A, 581B and the substantially straight segments 581G of the first connecting member 581C. The second connecting member 582C and the third connecting member 583C each have symmetric curvatures that form a mushroom shape. The second and third support frames 582, 583 include a pair of bends 582J, 583J at distal ends of the parallel segments 582A, 582B, 583A, 583B that direct the connecting member 582C, 583C away from the longitudinal axis L-L, a pair of bends 582H, 583H that turn distally and toward the longitudinal axis L-L, a pair of substantially straight segments 582G, 583G that extend toward the longitudinal axis L-L, and a bend 582F, 583F at the longitudinal axis L-L where the pair of substantially straight segments 582G, 583G converge. The pair of bends 583J of the third support frame 583 at distal ends of the parallel segments 583A, 583B bend toward the longitudinal axis L-L then curve distally, then curve away from the longitudinal axis L-L. The third connecting member 583C includes a proximal pair of substantially straight segments 583L that extend between bends 583J, 583H orthogonal to the longitudinal axis L-L.

The first connecting member 581C of the first support frame 581 has a maximum width W1 that is approximately equal to a width W4 measured orthogonal to the longitudinal axis L-L and between outer edges of the parallel segments 581A, 581B of the first support frame 581. Likewise, the first connecting member of the first loop member of the end effector 100 has a maximum width that is approximately equal to a width measured orthogonal to the longitudinal axis L-L and between outer edges of the first pair of spines.

The second connecting member 582C of the second support frame 582 has a maximum width W2 greater than a width W5 measured orthogonal to the longitudinal axis L-L and between outer edges of the parallel segments 582A, 582B of the second support frame 582. Likewise, the second connecting member of the second loop member has a second maximum width greater than a width measured orthogonal to the longitudinal axis and between outer edges of the second pair of spines.

The third connecting member 583C of the third support frame 583 has a maximum width W3 greater than a width W6 measured orthogonal to the longitudinal axis L-L and between outer edges of the parallel segments 583A, 583B of the third support frame 583. Likewise, the third connecting member of the third loop member has a third maximum width greater than a width measured orthogonal to the longitudinal axis and between outer edges of the third pair of spines. The maximum width W3 of the third connecting member 583C is greater than the width W5 measured between outer edges of the parallel segments 582A, 582B of the second support frame 582. Likewise, the third connecting member of the third loop member can have a third maximum width greater than a width measured orthogonal to the longitudinal axis between outer edges of the second pair of spines.

Each of the parallel segments can be approximately equal in length L3 to each other as measured parallel to the longitudinal axis L-L. Likewise, spines of the end effector can be approximately equal in length to each other as measured parallel to the longitudinal axis L-L.

Each of the support frames 581, 582, 583 can define a respective looped path having a cross-sectional shape orthogonal to the looped path that varies along the looped path. The cross-sectional shape can have a smaller area at bends 581K, 582K, 581H, 582J, 583J, 582H, 583H, 581F, 582F, 583F. For the second and third support frames 582, 583, the cross-sectional shape can have a smaller area in the over a majority of the looped path through the connecting members 582C, 583C compared to a majority of the looped path through the parallel segments 582A, 582B, 583A, 583B.

The end effector can include tubular housings over at least a portion of each support frame 581, 582, 583 including over a majority of each of the parallel segments 581A, 581B, 582A, 582B, 583A, 583B through each spine. Each spine can further include electrodes positioned over the tubular housings. Each spine can further include electrical conductors electrically connected to a respective electrode. The electrical conductors can extend through the tubular housings. Some or all of the tubular housings can include an irrigation lumen therethrough and irrigation ports.

The illustrated support frame assembly 580 includes three support frames 581, 582, 583. Alternatively, the support frame assembly 580 can include two support frames including an outer support frame configured similarly to the first support frame 581 and an inner support frame configured similarly to the second support frame 582, and third support frame 583.

FIGs. 7A and 7B are illustrations of a side and perspective view of a sixth example end effector configuration 600 of the end effector 100 in an unconstrained configuration. The sixth example end effector configuration 600 includes an outer loop member 601, an inner loop member 602, and a central loop member 603 that are symmetric about the longitudinal axis L-L. The loop members 601, 602, 603 are configured similarly to the loop members supported by the fifth support frame assembly 580 illustrated in FIG. 6 excepting some shape differences in connecting members 601C, 602C, 603C. The loop members 601, 602, 603 of the sixth example end effector configuration 600 include proximal segments 601D, 602D, 603D and spines 601A, 601B, 602A, 602B, 603A, 603B configured similarly to proximal segments and spines of the end effector supported by the fifth example support frame 580. Electrodes and irrigation ports are omitted from FIGs. 7A and 7B for the purposes of illustration.

The connecting members 601C, 602C, 603C are shaped to allow the end effector 600 to collapse to a low profile. The first connecting member 601C has a curved shape between the distal ends of the first pair of spines 601A, 601B. The second connecting member 602C and the third connecting member 603C each include a respective pair of curvatures 602F, 603F that extend from the distal ends of the second, third pair of spines 602A, 602B, 603A, 603B, away from the longitudinal axis L-L, turn distally, and turn toward the longitudinal axis L-L. The second connecting member 602C and the third connecting member 603C each include a curved shaped arc between the pair of curvatures 602F, 603F. At least a portion of each of the curved shapes of the first, second, and third connecting members 601C, 602C, 603C overlap each other. The curved shapes overlap at the distal vertex 50. The second and third connecting members 601C, 602C each include an acute bend 602H, 603H between the curvatures 602F, 603F and curved shaped arc.

The first connecting member 601C has a maximum width W1 that is approximately equal to a width W4 measured orthogonal to the longitudinal axis L-L and between outer edges of the first pair of spines 601A, 601B. The second connecting member 602C has a second maximum width W2 greater than a width W5 measured orthogonal to the longitudinal axis L-L and between outer edges of the second pair of spines 602A, 602B. The third connecting member 603C has a third maximum width W3 greater than a width W6 measured orthogonal to the longitudinal axis L-L and between outer edges of the third pair of spines 603A, 603B. The third maximum width W3 is approximately equal to the width W5 measured orthogonal to the longitudinal axis L-L and between outer edges of the second pair of spines 602A, 602B. Spines 601A, 601B, 602A, 602B, 603A, 603B are approximately equal in length L3 to each other as measured parallel to the longitudinal axis L-L.

The end effector configuration 600 can include electrodes, electrical conductors, and tubular housings similar to as described in relation to FIG. 6.

The illustrated end effector configuration 600 includes three loop members 601, 602, 603. Alternatively, the end effector configuration can include two loop members including an outer loop member configured similarly to the outer loop member 601 and an inner loop member configured similarly to the inner loop member 602 and central loop member 603.

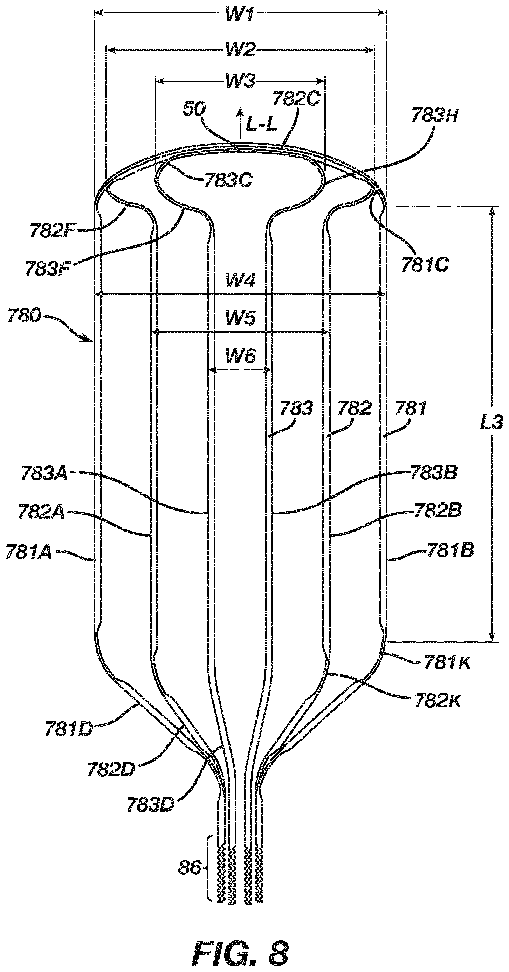

FIG. 8 is an illustration of a seventh example support frame assembly 780 of the end effector 100 in an unconstrained configuration. The seventh example support frame assembly 780 is similar to the support frame assembly of the end effector configuration 600 illustrated in FIGs. 7A and 7B except that a connecting member 783C of a third support frame 783 of the support frame assembly 780 illustrated in FIG. 8 has a pair of arcuate curvatures 783H rather than an acute bend 603H as illustrated in FIG. 7A.

The seventh example support frame assembly 780 includes a first support frame 781, a second support frame 782, and the third support frame 783. The support frames 781, 782, 783 overlap at a common distal vertex 50. The seventh example support frame assembly 780 includes proximal segments 781D, 782D, 783D and parallel segments 781A, 781B, 782A, 782B, 783A, 783B configured similarly to proximal segments and parallel segments of the fifth example support frame 580 illustrated in FIG. 6.

The first support frame 781 includes a first connecting member 781C having a curved shape between distal ends of a first pair of parallel segments 781A, 781B. The first connecting member 781C has a first maximum width W1 that is approximately equal to a width W4 measured orthogonal to the longitudinal axis L-L and between outer edges of the parallel segments 781A, 781B of the first support frame 781. Likewise, the first connecting member of the first loop member of the end effector 100 has a maximum width that is approximately equal to a width measured orthogonal to the longitudinal axis L-L and between outer edges of the first pair of spines.

The second support frame 782 includes a second connecting member 782C having a pair of curvatures 782F that extend from the distal ends of the parallel segments 782A, 782B, away from the longitudinal axis L-L, turn distally, and turn toward the longitudinal axis L-L. The second connecting member 782C has a curved shaped arc that between the pair of curvatures 782F. The second connecting member 782C of the second support frame 782 has a maximum width W2 greater than a width W5 measured orthogonal to the longitudinal axis L-L and between outer edges of the parallel segments 782A, 782B of the second support frame 782. Likewise, the second connecting member of the second loop member has a second maximum width greater than a width measured orthogonal to the longitudinal axis and between outer edges of the second pair of spines.

The third support frame 783 includes a third connecting member 783C having a pair of curvatures 783F that extend from the distal ends of the parallel segments 783A, 783B, away from the longitudinal axis L-L, turn distally, and turn toward the longitudinal axis L-L. The second connecting member 783C has a curved shaped arc that between the pair of curvatures 783F. The third connecting member 783C has a maximum width W3 greater than a width W6 measured orthogonal to the longitudinal axis L-L and between outer edges of the parallel segments 783A, 783B of the third support frame 783. Likewise, the third connecting member of the third loop member has a third maximum width greater than a width measured orthogonal to the longitudinal axis and between outer edges of the third pair of spines. The maximum width W3 of the third connecting member 783C is approximately equal to and/or less than the width W5 measured between outer edges of the parallel segments 782A, 782B of the second support frame 782. Likewise, the third connecting member of the third loop member can have a third maximum width approximately equal to and/or less than a width measured orthogonal to the longitudinal axis between outer edges of the second pair of spines.

Each of the parallel segments 781A, 781B, 782A, 782B, 783A, 783B can be approximately equal in length L3 to each other as measured parallel to the longitudinal axis L-L. Likewise, spines of the end effector can be approximately equal in length to each other as measured parallel to the longitudinal axis L-L.

Each of the support frames 781, 782, 783 can define a respective looped path having a cross-sectional shape orthogonal to the looped path that varies along the looped path. The cross-sectional shape can have a smaller area at bends 781K, 782K. The cross-sectional shape can have a smaller area in the over a majority of the looped path through the connecting members 781C, 782C, 783C compared to a majority of the looped path through the parallel segments 781A, 781B, 782A, 782B, 783A, 783B.

The end effector can include tubular housings over at least a portion of each support frame 781, 782, 783 including over a majority of each of the parallel segments 781A, 781B, 782A, 782B, 783A, 783B through each spine. Each spine can further include electrodes positioned over the tubular housings. Each spine can further include electrical conductors electrically connected to a respective electrode. The electrical conductors can extend through the tubular housings. Some or all of the tubular housings can include an irrigation lumen therethrough and irrigation ports.

The illustrated support frame assembly 780 includes three support frames 781, 782, 783. Alternatively, the support frame assembly 780 can include two support frames including an outer support frame configured similarly to the first support frame 781 and an inner support frame configured similarly to the second support frame 782, and third support frame 783.

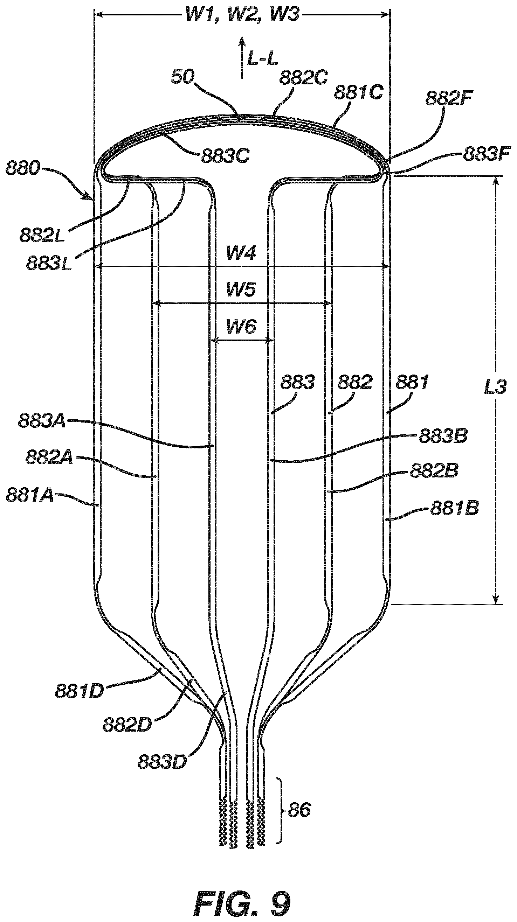

FIG. 9 is an illustration of an eighth example support frame assembly 880 of the end effector 100 in an unconstrained configuration. The eight example support frame assembly 880 is similar to the seventh example support frame assembly 780 illustrated in FIG. 8 except that a second connecting member 882C and a third connecting member 883C of the eight example support frame assembly 880 are wider than corresponding connecting members 782C, 783C of the seventh example support frame assembly 780.

The eighth example support frame assembly 880 includes a first support frame 881, a second support frame 882, and the third support frame 883. The support frames 881, 882, 883 overlap at a common distal vertex 50. The eighth example support frame assembly 880 includes proximal segments 881D, 882D, 883D and parallel segments 881A, 881B, 882A, 882B, 883A, 883B configured similarly to proximal segments and parallel segments of the seventh example support frame 780 illustrated in FIG. 8.

The first support frame 881 includes a first connecting member 881C having a curved shape between distal ends of a first pair of parallel segments 881A, 881B. The first connecting member 881C has a first maximum width W1 that is approximately equal to a width W4 measured orthogonal to the longitudinal axis L-L and between outer edges of the parallel segments 881A, 881B of the first support frame 881. Likewise, the first connecting member of the first loop member of the end effector 100 has a maximum width that is approximately equal to a width measured orthogonal to the longitudinal axis L-L and between outer edges of the first pair of spines.

The second support frame 882 includes a second connecting member 882C having a pair of substantially straight segments 882L that extend from distal ends of the parallel segments 882A, 882B away from the longitudinal axis and orthogonal to the longitudinal axis L-L. The second connecting member 882C includes curvatures 882F that extend from the substantially straight segments 882L, turn distally, and turn toward the longitudinal axis L-L. The second connecting member 882C has a curved shaped arc that between the pair of curvatures 882F. The second connecting member 882C of the second support frame 882 has a maximum width W2 greater than a width W5 measured orthogonal to the longitudinal axis L-L and between outer edges of the parallel segments 882A, 882B of the second support frame 882. Likewise, the second connecting member of the second loop member has a second maximum width greater than a width measured orthogonal to the longitudinal axis and between outer edges of the second pair of spines. The maximum width W2 of the second connecting member 882C is approximately equal to the width W4 measured orthogonal to the longitudinal axis L-L and between outer edges of the parallel segments 881A, 881B of the first support frame 881. Likewise, the first connecting member of the first loop member of the end effector can have a maximum width that is approximately equal to a width measured orthogonal to the longitudinal axis between outer edges of the first pair of spines.

The third support frame 883 includes a third connecting member 883C having a pair of substantially straight segments 883L that extend from distal ends of the parallel segments 883A, 883B away from the longitudinal axis and orthogonal to the longitudinal axis L-L. The third connecting member 883C includes curvatures 883F that extend from the substantially straight segments 883L, turn distally, and turn toward the longitudinal axis L-L. The third connecting member 883C has a curved shaped arc that between the pair of curvatures 883F. The third connecting member 883C has a maximum width W3 greater than a width W6 measured orthogonal to the longitudinal axis L-L and between outer edges of the parallel segments 883A, 883B of the third support frame 883. Likewise, the third connecting member of the third loop member has a third maximum width greater than a width measured orthogonal to the longitudinal axis and between outer edges of the third pair of spines. The maximum width W3 of the third connecting member 883C is approximately equal to and/or less than the width W5 measured between outer edges of the parallel segments 881A, 881B of the first support frame 881. Likewise, the third connecting member of the third loop member can have a third maximum width approximately equal to and/or less than a width measured orthogonal to the longitudinal axis between outer edges of the first pair of spines.

Each of the parallel segments 881A, 881B, 882A, 882B, 883A, 883B can be approximately equal in length L3 to each other as measured parallel to the longitudinal axis L-L. Likewise, spines of the end effector can be approximately equal in length to each other as measured parallel to the longitudinal axis L-L.

Each of the support frames 881, 882, 883 can define a respective looped path having a cross-sectional shape orthogonal to the looped path that varies along the looped path. The cross-sectional shape can have a smaller area at bends 881K, 882K. The cross-sectional shape can have a smaller area in the over a majority of the looped path through the connecting members 881C, 882C, 883C compared to a majority of the looped path through the parallel segments 881A, 881B, 882A, 882B, 883A, 883B.

The end effector can include tubular housings over at least a portion of each support frame 881, 882, 883 including over a majority of each of the parallel segments 881A, 881B, 882A, 882B, 883A, 883B through each spine. Each spine can further include electrodes positioned over the tubular housings. Each spine can further include electrical conductors electrically connected to a respective electrode. The electrical conductors can extend through the tubular housings. Some or all of the tubular housings can include an irrigation lumen therethrough and irrigation ports.

The illustrated support frame assembly 880 includes three support frames 881, 882, 883. Alternatively, the support frame assembly 880 can include two support frames including an outer support frame configured similarly to the first support frame 881 and an inner support frame configured similarly to the second support frame 882, and third support frame 883.

## Claims

1. An apparatus comprising:
an elongated shaft (9) comprising a proximal portion (12) and a distal portion (14A), the elongated shaft configured to be manipulated at the proximal portion to position the distal portion into the heart of a patient, the elongated shaft defining a longitudinal axis of the apparatus; and
an end effector (100) disposed proximate the distal portion of the elongated shaft, the end effector movable from a constrained configuration sized to traverse a catheter to an approximately planar unconstrained configuration, the end effector comprising six spines (1A, 1B, 2A, 2B, 3A, 3B) and three connecting members (1C, 2C, 3C),
each of the six spines being approximately parallel to the longitudinal axis and comprising electrodes (37) thereon,
each of the three connecting members joining distal ends of two respective spines of the six spines,
the three connecting members comprising a central connecting member (182C) approximately symmetric to the longitudinal axis and two outer connecting members (181C, 183C) asymmetric to the longitudinal axis, and
**characterised by** each of the two outer connecting members comprising a respective pair of substantially straight segments (181F, 181H, 183F, 183H) connected by a respective bend (181J, 183J) that is more acute in the constrained configuration compared to the unconstrained configuration.

2. The apparatus of claim 1,
the three connecting members overlapping at a distal vertex (50) of the end effector, the distal vertex being aligned with the longitudinal axis.

3. The apparatus of claim 2, the end effector further comprising:
a mechanical linkage binding the three connecting members at the distal vertex.

4. The apparatus of claim 2,
each of the respective pair of substantially straight segments respectively comprising a first straight segment (181F, 183F) and a second straight segment (181H, 183H), the first straight segment comprising a first length measured from the distal vertex to the respective bend, and the second straight segment comprising a second length measured from the bend to the distal end of the respective spine.

5. The apparatus of claim 4, the first length being less than the second length.

6. The apparatus of claim 4, the first length being approximately equal to the second length.

7. The apparatus of claim 1,
the central connecting member comprising three substantially straight segments (382H, 382F) and two bends (382J), the two bends each being more acute in the constrained configuration compared to the unconstrained configuration.

8. The apparatus of claim 1, the end effector further comprising:
two outer loop members (1, 3) each respectively comprising a respective outer connecting member of the two outer connecting members and the two respective spines joined to the respective outer connecting member;
two outer support frames (181, 183) each joined to the distal portion of the elongated shaft and extending through a respective outer loop member of the two outer loop members;
a central loop member (2) comprising the central connecting member and the two respective spines joined to the central connecting member; and
a central support frame (182) joined to the distal portion of the elongated shaft and extending through the central loop member.

9. The apparatus of claim 8, each of the six spines comprising a respective tubular housing, each of the outer support frame and the central support frame extending through respective tubular housings, and electrodes of each of the six spines being disposed over the respective tubular housings.

10. The apparatus of claim 9, each of the six spines comprising electrical conductors each electrically connected to a respective electrode and extending through at least a portion of the respective tubular housing.

11. The apparatus of claim 9, at least a portion of the respective tubular housings each comprising an irrigation lumen therethrough and irrigation ports.

12. The apparatus of claim 1, further comprising at least one pull wire extending through the elongated shaft and attached to the distal portion of the elongated shaft so that when the pull wire is retracted toward the proximal portion relative to the elongated shaft, the distal portion and the end effector are bent at an angle with respect to the longitudinal axis.

13. The apparatus of claim 1, the end effector being sized to collapse within a 10 French sheath.

## Patentansprüche

1. Einrichtung, umfassend:
einen länglichen Schaft (9), umfassend einen proximalen Abschnitt (12) und einen distalen Abschnitt (14A), wobei der längliche Schaft konfiguriert ist, um an dem proximalen Abschnitt manipuliert zu werden, um den distalen Abschnitt in ein Herz eines Patienten zu positionieren, wobei der längliche Schaft eine Längsachse der Einrichtung definiert; und
einen Endeffektor (100), der nahe dem distalen Abschnitt des länglichen Schafts angeordnet ist, wobei der Endeffektor von einer eingeschränkten Konfiguration, die bemessen ist, um einen Katheter zu durchqueren, in eine etwa planare uneingeschränkte Konfiguration bewegbar ist, der Endeffektor umfassend sechs Streben (1A, 1B, 2A, 2B, 3A, 3B) und drei Verbindungselemente (1C, 2C, 3C),
wobei jede der sechs Streben etwa parallel zu der Längsachse ist und Elektroden (37) daran umfasst,
wobei jedes der drei Verbindungselemente distale Enden von zwei jeweiligen Streben der sechs Streben vereint,
die drei Verbindungselemente umfassend ein zentrales Verbindungselement (182C) etwa symmetrisch zu der Längsachse und zwei äußere Verbindungselemente (181C, 183C) asymmetrisch zu der Längsachse, und
**dadurch gekennzeichnet, dass** jedes der zwei äußeren Verbindungselemente ein jeweiliges Paar von im Wesentlichen geraden Segmenten (181F, 181H, 183F, 183H) umfasst, die durch eine jeweilige Biegung (181J, 183J) verbunden sind, die in der eingeschränkten Konfiguration im Vergleich zu der uneingeschränkten Konfiguration spitzer ist.

2. Einrichtung nach Anspruch 1,
wobei die drei Verbindungselemente sich an einem distalen Scheitelpunkt (50) des Endeffektors überlappen, der distale Scheitelpunkt mit der Längsachse ausgerichtet ist.

3. Einrichtung nach Anspruch 2, der Endeffektor ferner umfassend:
eine mechanische Verknüpfung, die die drei Verbindungselemente an dem distalen Scheitelpunkt bindet.

4. Einrichtung nach Anspruch 2,
jedes des jeweiligen Paars von im Wesentlichen geraden Segmenten jeweils umfassend ein erstes gerades Segment (181F, 183F) und ein zweites gerades Segment (181H, 183H), das erste gerade Segment umfassend eine erste Länge, die von dem distalen Scheitelpunkt zu der jeweiligen Biegung gemessen wird, und das zweite gerade Segment umfassend eine zweite Länge, die von der Biegung zu dem distalen Ende der jeweiligen Strebe gemessen wird.

5. Einrichtung nach Anspruch 4, wobei die erste Länger kürzer als die zweite Länge ist.

6. Einrichtung nach Anspruch 4, wobei die erste Länge etwa gleich der zweiten Länge ist.

7. Einrichtung nach Anspruch 1,
das zentrale Verbindungselement umfassend drei im Wesentlichen gerade Segmente (382H, 382F) und zwei Biegungen (382J), wobei die zwei Biegungen in der eingeschränkten Konfiguration im Vergleich zu der uneingeschränkten Konfiguration jeweils spitzer sind.

8. Einrichtung nach Anspruch 1, der Endeffektor ferner umfassend:
zwei äußere Schlaufenelemente (1, 3), jeweils umfassend ein jeweiliges äußeres Verbindungselement der zwei äußeren Verbindungselemente und die zwei jeweiligen Streben, die mit dem jeweiligen äußeren Verbindungselement vereint sind;
zwei äußere Stützrahmen (181, 183), die jeweils mit dem distalen Abschnitt des länglichen Schafts vereint sind und sich durch ein jeweiliges äußeres Schlaufenelement der zwei äußeren Schlaufenelemente erstrecken;
ein zentrales Schlaufenelement (2), umfassend das zentrale Verbindungselement und die zwei jeweiligen Streben, die mit dem zentralen Verbindungselement vereint sind; und
einen zentralen Stützrahmen (182), der mit dem distalen Abschnitt des länglichen Schafts vereint ist und sich durch das zentrale Schlaufenelement erstreckt.

9. Einrichtung nach Anspruch 8, jede der sechs Streben umfassend ein jeweiliges rohrförmiges Gehäuse, wobei sich der äußere Stützrahmen und der zentrale Stützrahmen jeweils durch jeweilige rohrförmige Gehäuse erstrecken und Elektroden jeder der sechs Streben über den jeweiligen rohrförmigen Gehäusen angeordnet sind.

10. Einrichtung nach Anspruch 9, jede der sechs Streben umfassend elektrische Leiter, die jeweils mit einer jeweiligen Elektrode elektrisch verbunden sind und sich durch mindestens einen Abschnitt des jeweiligen rohrförmigen Gehäuses erstrecken.

11. Einrichtung nach Anspruch 9, mindestens ein Abschnitt der jeweiligen rohrförmigen Gehäuse jeweils umfassend ein Spüllumen dort hindurch und Spülöffnungen.

12. Einrichtung nach Anspruch 1, ferner umfassend mindestens einen Zugdraht, der sich durch den länglichen Schaft erstreckt und an dem distalen Abschnitt des länglichen Schafts so angebracht ist, dass, wenn der Zugdraht zu dem proximalen Abschnitt hin relativ zu dem länglichen Schaft zurückgezogen ist, der distale Abschnitt und der Endeffektor in einem Winkel bezüglich der Längsachse gebogen sind.

13. Einrichtung nach Anspruch 1, wobei der Endeffektor so bemessen ist, dass er in einer 10-French-Schleuse zusammenklappt.

## Revendications

1. Appareil comprenant :
une tige allongée (9) comprenant une partie proximale (12) et une partie distale (14A), la tige allongée étant conçue pour être manipulée au niveau de la partie proximale pour positionner la partie distale dans le cœur d'un patient, la tige allongée définissant un axe longitudinal de l'appareil ; et
un effecteur terminal (100) disposé à proximité de la partie distale de la tige allongée, l'effecteur terminal étant déplaçable d'une configuration contrainte dimensionnée pour traverser un cathéter à une configuration non contrainte approximativement plane, l'effecteur terminal comprenant six éléments d'armature (1A, 1B, 2A, 2B, 3A, 3B) et trois éléments de liaison (1C, 2C, 3C),
chacun des six éléments d'armature étant approximativement parallèle à l'axe longitudinal et comprenant des électrodes (37) sur ceux-ci,
chacun des trois éléments de liaison joignant des extrémités distales de deux éléments d'armature respectifs des six éléments d'armature,
les trois éléments de liaison comprenant un élément de liaison central (182C) approximativement symétrique par rapport à l'axe longitudinal et deux éléments de liaison externes (181C, 183C) asymétriques par rapport à l'axe longitudinal, et
**caractérisé par** chacun des deux éléments de liaison externes comprenant une paire respective de segments sensiblement rectilignes (181F, 181H, 183F, 183H) reliés par un cintrage (181J, 183J) respectif qui est plus aigu dans la configuration contrainte par comparaison avec la configuration sans contrainte.

2. Appareil selon la revendication 1,
les trois éléments de liaison se chevauchant au niveau d'un sommet distal (50) de l'effecteur terminal, le sommet distal étant aligné sur l'axe longitudinal.

3. Appareil selon la revendication 2, l'effecteur terminal comprenant en outre :
une liaison mécanique liant les trois éléments de liaison au niveau du sommet distal.

4. Appareil selon la revendication 2,
chacun parmi la paire respective de segments sensiblement rectilignes comprenant respectivement un premier segment rectiligne (181F, 183F) et un second segment rectiligne (181H, 183H), le premier segment rectiligne comprenant une première longueur mesurée du sommet distal au cintrage respectif, et le second segment rectiligne comprenant une seconde longueur mesurée du cintrage à l'extrémité distale de l'élément d'armature respectif.

5. Appareil selon la revendication 4, la première longueur étant inférieure à la seconde longueur.

6. Appareil selon la revendication 4, la première longueur étant approximativement égale à la seconde longueur.

7. Appareil selon la revendication 1,
l'élément de liaison central comprenant trois segments sensiblement rectilignes (382H, 382F) et deux cintrages (382J), chacun des deux cintrages étant plus aigu dans la configuration contrainte par comparaison avec la configuration sans contrainte.

8. Appareil selon la revendication 1, l'effecteur terminal comprenant en outre :
deux éléments de boucle externes (1, 3) comprenant chacun respectivement un élément de liaison externe respectif des deux éléments de liaison externes et les deux éléments d'armature respectifs joints à l'élément de liaison externe respectif ;
deux cadres de support externes (181, 183) joints chacun à la partie distale de la tige allongée et s'étendant à travers un élément de boucle externe respectif des deux éléments de boucle externes ;
un élément de boucle central (2) comprenant l'élément de liaison central et les deux éléments d'armature respectifs joints à l'élément de liaison central ; et
un cadre de support central (182) joint à la partie distale de la tige allongée et s'étendant à travers l'élément de boucle central.

9. Appareil selon la revendication 8, chacun des six éléments d'armature comprenant un logement tubulaire respectif, chacun du cadre de support externe et du cadre de support central s'étendant à travers des logements tubulaires respectifs, et des électrodes de chacun des six éléments d'armature étant disposées par-dessus les logements tubulaires respectifs.

10. Appareil selon la revendication 9, chacun des six éléments d'armature comprenant des conducteurs électriques connectés chacun électriquement à une électrode respective et s'étendant à travers au moins une partie du logement tubulaire respectif.

11. Appareil selon la revendication 9, au moins une partie des logements tubulaires respectifs comprenant chacun une lumière d'irrigation à travers celle-ci et des orifices d'irrigation.

12. Appareil selon la revendication 1, comprenant en outre au moins un fil de traction s'étendant à travers la tige allongée et fixé à la partie distale de la tige allongée de sorte que lorsque le fil de traction est rétracté en direction de la partie proximale par rapport à la tige allongée, la partie distale et l'effecteur terminal sont cintrés selon un angle par rapport à l'axe longitudinal.

13. Appareil selon la revendication 1, l'effecteur terminal étant dimensionné pour se replier à l'intérieur d'une gaine de 10 French.
